# EUROPEAN PATENT APPLICATION

(11) **EP 4 159 104 A1**
(43) Date of publication of application: **05.04.2023**
(21) Application number: 21817700.4
(22) Date of filing: 28.05.2021
(51) Int. Cl.: A61B 1/00, A61B 5/00

(54) **MEDICAL CAPSULE**

(30) Priority: 01.06.2020 CN 202010486154
(71) Applicant: Ankon Medical Technologies (Shanghai) Co., Ltd, Shanghai 200131 (CN)
(72) Inventor: LIU, Lei, Shanghai 201206 (CN); DUAN, Xiaodong, Shanghai 201206 (CN)
(74) Representative: Lavoix
(86) International application number: PCT/CN2021/096831
(87) International publication number: WO 2021/244431

(57) **Abstract**

A medical capsule, comprising an enclosure (1), the enclosure (1) comprising an opening at one end along an axial direction; a PCB group (2) and a functional unit which are arranged inside the enclosure (1); wherein the PCB group (2) comprises a plurality of PCBs which are connected through flexible printed circuits (20) and are spaced from each other, and the functional unit comprises a battery unit (6) electrically connected to the PCB; and a pressure detection unit (3), comprising a pressure transmission film (30) assembled at the opening, a pressure sensor (31) positioned in the enclosure (1), and a pressure collecting and processing circuit board (32) electrically connected to the pressure sensor (31), wherein the pressure collecting and processing circuit board (32) is connected to one of the PCBs through a flexible printed circuit (20). The medical capsule is simple in structure, easy to equip and allows for expansion of functions as needed.

## Description

### FIELD OF INVENTION

The present invention relates to a medical device, and more particularly to a medical capsule.

### BACKGROUND

With the wide application of capsule endoscope, more and more medical wireless capsule devices are developed and put into clinical use, and functions of the medical wireless capsule devices are gradually increased and improved. Pressure measurement in the gastrointestinal tract (GI) is clinically important, so a pressure capsule endoscope is developed for non-invasive and comfortable measurement of pressures in the GI tract, to provide a basis for clinical diagnosis.

In the prior art of the pressure capsule endoscope, either a flexible enclosure filled with liquid therein is used, which has a very complex structure and is extremely difficult to produce and assemble, or the method of measuring pressure for the evaluation of the efficacy of vibration therapy is disclosed, but not disclosing a specific assembly structure of the pressure capsule endoscope, and not providing a technical solution for solving the problems of complex structure and high assembly difficulty of the pressure capsule endoscope.

Therefore, it is necessary to improve existing medical capsule to solve the above problems.

### SUMMARY OF THE INVENTION

An object of the present invention is to provide a medical capsule which is simple in structure, easy to equip and allows for expansion of functions as needed.

In order to achieve the object, the present invention adopts a technical solution as follows:
a medical capsule comprising:
an enclosure, comprising an opening at one end along an axial direction;
a PCB group and a functional unit which are arranged inside the enclosure, wherein the PCB group comprises a plurality of PCBs which are connected through flexible printed circuits and are spaced from each other; and wherein the functional unit comprises a battery unit electrically connected to the PCB;
a pressure detection unit, comprising a pressure transmission film assembled at the opening, a pressure sensor positioned in the enclosure, and a pressure collecting and processing circuit board electrically connected to the pressure sensor, wherein the pressure collecting and processing circuit board is connected to one of the PCBs through a flexible printed circuit.

Further, the pressure transmission film comprises a pressure groove which is concavely formed from inside to outside and a pressure film which is arranged on the bottom surface of the pressure groove, and the pressure sensor is housed in the pressure groove and faces the pressure film.

Further, the enclosure comprises a front enclosure and a middle enclosure adapted to each other, and a plurality of supporting legs are convexly fitted inside one end of the middle enclosure away from the front enclosure. The pressure collecting and processing circuit board is fixed on one side of the support legs away from the front enclosure, the pressure transmission film is assembled at one end of the middle enclosure away from the front enclosure, and the pressure sensor is positioned between the pressure transmission film and the pressure collecting and processing circuit board.

Further, the periphery of the pressure collecting and processing circuit board is convexly fitted with fixing legs corresponding to the plurality of supporting legs one by one.

Further, the enclosure comprises a front enclosure and a middle enclosure adapted to each other, and a fixing groove positioned at the joint of the front enclosure and the middle enclosure.

The PCB group further comprises fixing structures for connecting two adjacent PCBs, wherein the edge of one of the fixing structures is fixed in the fixing groove to fix the PCB group in the enclosure.

Further, the PCB group comprises an illumination circuit board, an image capturing and processing board, a power supply board and an antenna transceiver board which are sequentially arranged. The fixing structures comprise an illumination fixing structure, and the illumination circuit board is located in the illumination fixing structure. The image capturing and processing board is fixed on one side of the illumination fixing structure away from the front enclosure. The edge of the illumination fixing structure is housed in the fixing groove.

Further, the functional unit further comprises an illumination unit, a camera unit and an antenna, wherein the illumination unit is fixed on one side of the illumination circuit board facing the front enclosure, the camera unit is fixed on one side of the image capturing and processing board facing the front enclosure, the battery unit is electrically connected to the power supply board, and the antenna is fixed on the antenna transceiver board. The illumination fixing structure is annular, and the illumination circuit board is provided with a through hole for the camera unit to pass through towards the front enclosure.

Further, the illumination fixing structure comprises an annular retaining wall, a retaining frame protruding outwards from a front end of part of the retaining wall, and a plurality of fixing clamps protruding inwards from the retaining wall to fix and limit the illumination circuit board, wherein an edge of the retaining frame is fixed in the fixing groove.

The illumination circuit board is housed in a mounting groove formed by the surrounding of the retaining wall, and the image capturing and processing board is fixed to one side of the retaining wall away from the front enclosure.

Further, the battery unit is disposed between the power supply board and the antenna transceiver board, and the fixing structure further comprises battery fixing plates respectively located at the positive and negative electrodes of the battery unit, and the two battery fixing plates are fixedly connected to the power supply board and the antenna transceiver board respectively.

Further, the battery unit comprises a plurality of batteries connected in series, battery connectors electrically connected between the adjacent batteries, and electrode connectors connected to the positive and negative ends of the battery unit, and the two electrode connectors are electrically connected to the power supply board and the antenna transceiver board respectively.

Further, the medical capsule further comprises a magnet unit disposed between the image capturing and processing board and the power supply board. The magnet unit comprises a magnet and a magnet fixing cover sleeved on the magnet, and the magnet fixing cover is fixedly connected to the image capturing and processing board and the power supply board.

The present invention has advantageous effects as compared with the prior art. Specifically, an endoscope imaging module is constituted by the PCB group and the functional unit, and pressure detection of the GI tract is realized by the pressure detection unit, so that the functions of the medical capsule are diversified. In addition, the pressure detection unit is disposed on the opening side of the enclosure, so that the internal structure of the medical capsule is reasonably distributed, the assembly is quick, and mass production is facilitated. Furthermore, the present invention has the feature of easy expansion and extension of functions. When different functions need to be added, it is convenient to expand the functions of the medical capsule by simply adding PCB, functional components, and corresponding fixing pieces in sequence.

### RRIEF DESCRIPTION OF THE DRAWINGS

In order to illustrate more clearly the technical solutions in the embodiments or prior art of the invention, the following is a brief description of the accompanying drawings to be used in the description of the embodiments or prior art. It is obvious that the accompanying drawings in the following description are only embodiments of the invention and that other accompanying drawings can be obtained without creative work for those ordinary skilled in the art on the basis of the accompanying drawings provided.
FIG. 1 is a schematic view showing the structure of a medical capsule according to the present invention.
FIG. 2 is a schematic view showing the structure of a pressure transmission film in the medical capsule according to the present invention.
FIG. 3 is a schematic view showing the structure of a pressure sensor and a pressure collecting and processing circuit board in the medical capsule according to the present invention.
FIG. 4 is a schematic view showing the structure of a PCB group in the medical capsule according to the present invention.
FIG. 5 is a schematic view showing the assembly structure of a PCB group, an illumination fixing structure, a magnet fixing cover and battery fixing plates in the medical capsule according to the present invention.
FIG. 6 is a schematic view showing the assembly structure of an illumination circuit board and an illumination fixing structure in the medical capsule according to the present invention.
FIG. 7 is a schematic view showing the assembly of the medical capsule with the enclosure through the illumination fixing structure according to the present invention.

100-medical capsule, 1-enclosure, 10-front enclosure, 11-middle enclosure, 13-supporting leg, 130-gap, 14-fixing groove, 12-illumination fixing structure, 120-retaining wall, 121-retaining frame, 122-fixing clamp, 123-mounting groove, 124-positioning pin, 125-positioning hole, 2-PCB group, 20-flexible printed circuit, 210-illumination circuit board, 2100-through hole, 211-image capturing and processing board, 212-power supply board, 2120-notch, 213-antenna transceiver board, 3-pressure detection unit, 30-pressure transmission film, 301-pressure groove, 302-pressure film, 303-annular step, 304-bonding part, 31-pressure sensor, 310-fixing seat, 32-pressure collecting and processing circuit board, 320-fixing leg, 4-magnet unit, 40-magnet fixing cover, 41-dispensing hole, 5-camera unit, 6-battery unit, 60-battery, 61-battery connector, 62-electrode connector, 63-insulation gasket, 64-battery fixing plate, 7-antenna.

### DETAILED DESCRIPTION

The present invention is described in detail below with reference to the accompanying drawings and preferred embodiments. However, the embodiments are not intended to limit the invention, and the structural, method, or functional changes made by those skilled in the art in accordance with the embodiments are comprised in the scope of the present invention.

In the figures of the present inve-ntion, some sizes of a structure or portion may be exaggerated relative to other structures or portions for ease of illustration, and thus, are merely used to illustrate the basic structure of the subject matter of the present invention.

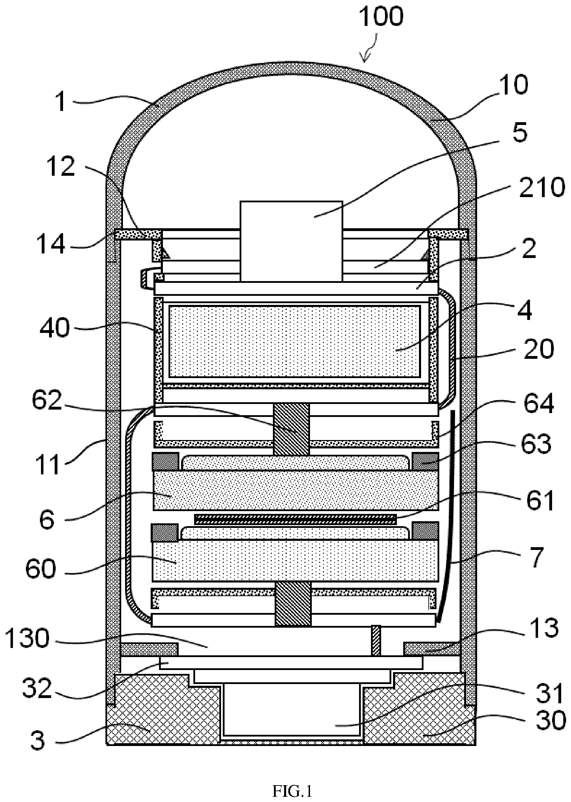

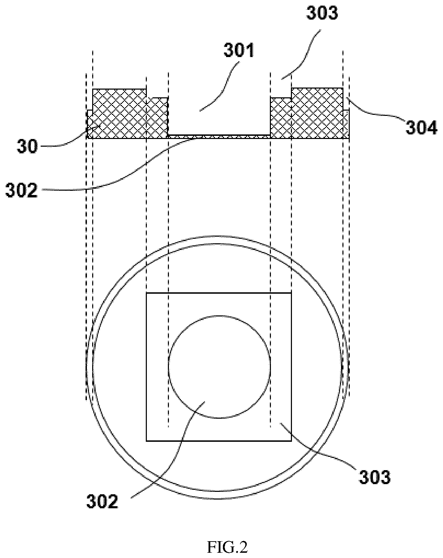

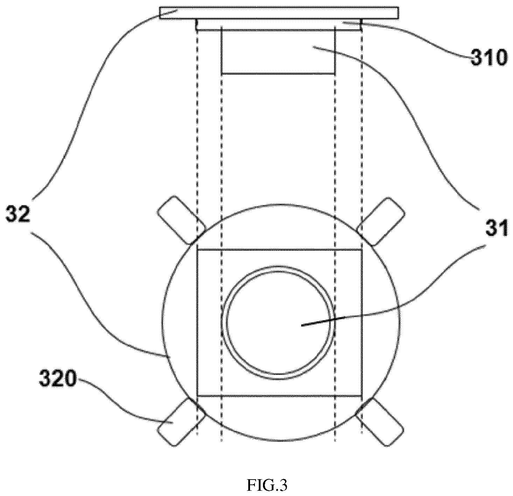

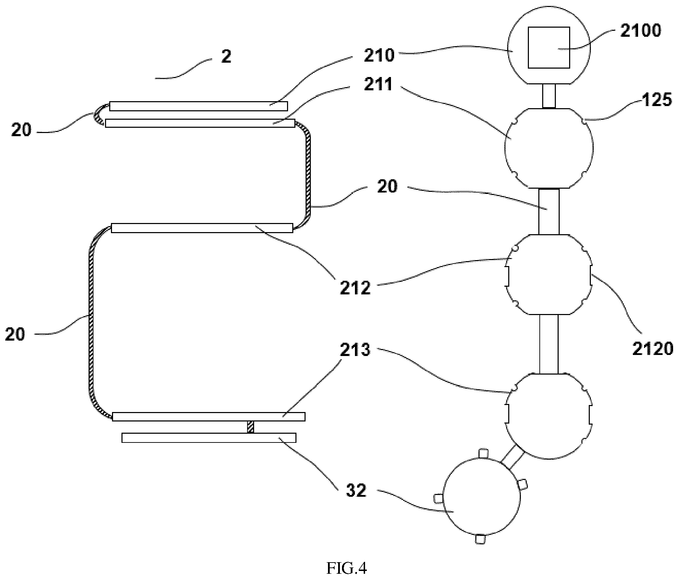

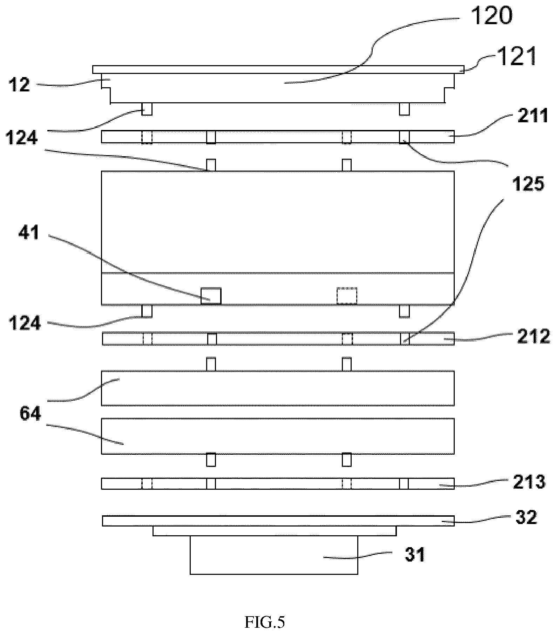

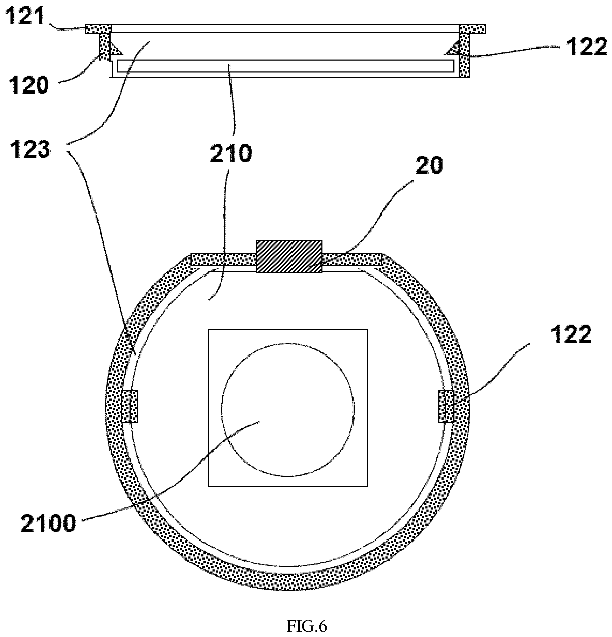

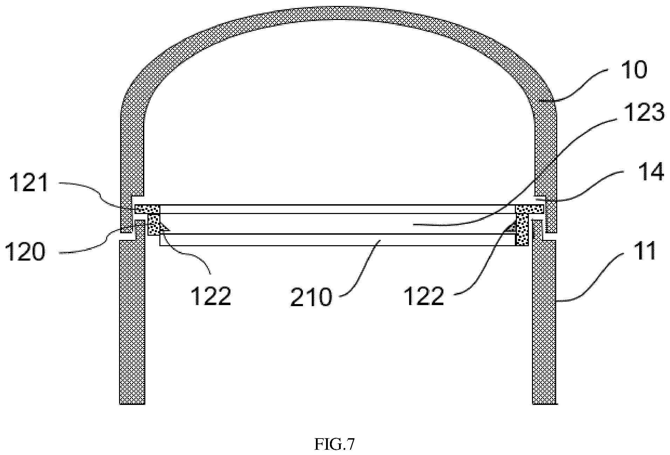

Referring to FIG. 1 to FIG. 7, showing a medical capsule 100 according to a preferred embodiment of the present invention. The medical capsule 100 comprises an enclosure 1, a PCB (Printed Circuit Board) group 2, a functional unit, a pressure detection unit 3, and a magnet unit 4. The PCB group 2, the functional unit, the pressure detection unit 3, and the magnet unit 4 are arranged inside the enclosure 1.

The enclosure 1 is biocompatible and is not corroded by digestive fluid. It can be made still using the material of the enclosure in an existing capsule endoscope, or designed separately. In addition, the enclosure 1 is constructed by at least two parts joined together to facilitate fixing other structures in the enclosure 1.

Specifically, the enclosure 1 comprises an opening located at one end thereof along an axial direction, and the pressure detection unit 3 is installed at the opening, so that internal structures of the medical capsule 100 is reasonably distributed, the assembly is quick, and the mass production is facilitated.

In the embodiment shown in FIG. 1 and FIG. 4 to FIG. 7, the enclosure 1 specifically comprises a front enclosure 10 and a middle enclosure 11 adapted to each other, the front enclosure 10 and the middle enclosure 11 are connected by adhesive or laser welding. A fixing groove 14 is formed at the joint of the front enclosure 10 and the middle enclosure 11. The opening is set at an end of the middle enclosure 11 away from the front enclosure 10, and a plurality of supporting legs 13 are convexly arranged inside the end of the middle enclosure 11 away from the front enclosure 10. The fixing groove 14 and the supporting legs 13 are so arranged that the PCB group 2 and the pressure detection unit 3 can be easily installed.

The PCB group 2 comprises a plurality of PCBs connected by flexible printed circuits 20 and spaced apart from each other. Further, the PCB group 2 further comprises fixing structures for connecting two adjacent PCBs, wherein the edge of one of the fixing structures is fixed in the fixing groove 14 to fix the PCB group 2 in the enclosure 1.

One of the fixing structures and the adjacent PCB is provided with a positioning pin 124, and the other is provided with a positioning hole 125, so that the rotation between the two along circumferential direction can be prevented. Preferably, the positioning pin 124 is disposed on the fixing structure, the positioning hole 125 is cut in the PCB, and it is simple to make this.

Specifically, the plurality of PCBs comprise an illumination circuit board 210, an image capturing and processing board 211, a power supply board 212 and an antenna transceiver board 213 which are sequentially arranged.

In the embodiment as shown in FIG. 1 and FIG. 5 to FIG. 7, the fixing structures comprise an illumination fixing structure 12, the illumination circuit board 210 is located in the illumination fixing structure 12, and the image capturing and processing board 211 is fixed on a side of the illumination fixing structure 12 away from the front enclosure 10. The edge of the illumination fixing structure 12 is housed in the fixing groove 14 and is fixed to the enclosure 1 by glue.

The illumination fixing structure 12 comprises an annular retaining wall 120, a retaining frame 121 protruding outwards from a front end of a portion of the retaining wall 120 for being housed in the fixing groove 14, a plurality of fixing clamps 122 protruding inwards from the retaining wall 120 for fixing and limiting the illumination circuit board 210, and a notch disposed on the retaining wall 120 for the flexible printed circuit 20 to pass through. The retaining wall 120 is enclosed to form a mounting groove 123.

The fixing clamps 122 comprise two groups of fixing clamps 122 spaced apart along the front-rear direction of the medical capsule 100 to clamp the illumination circuit board 210, wherein one group of fixing clamps 122 located at the front side comprises a plurality of clamping buckles spaced apart, and the other one group of fixing clamps 122 located at the rear side comprises clamping strips extending along the retaining wall 120. Preferably, the clamping buckles and the clamping strips are installed in a staggered form, the illumination circuit board 210 is received in the mounting groove 123 and clamped between the two groups of fixing clamps 122, and the flexible printed circuit 20 connected to the illumination circuit board 210 extends out through the notch to connect to the image capturing and processing board 211.

Positioning pins 124 and/or positioning holes 125 are arranged on the side of the retaining wall 120 away from the front enclosure 10, and accordingly matching positioning holes 125 and/or positioning pins 124 are arranged on the image capturing and processing board 211. The image capturing and processing board 211 and the retaining wall 120 are fixed to each other by the positioning pins 124 and the positioning holes 125, which can prevent rotation along the circumferential direction. Preferably, the retaining wall 120 is also provided with a dispensing groove in a side thereof away from the front enclosure 10 for dispensing glue to improve the stability of the connection between the retaining wall 120 and the front enclosure 10.

After the plurality of PCBs are connected into a whole by the fixing structures and the flexible printed circuits 20, the edge of the retaining frame 121 is fixed in the fixing groove 14, so that the PCB group 2 can be integrally fixed in the enclosure 1, which is convenient to assemble.

The functional units comprise an illumination unit (not shown), a camera unit 5, a battery unit 6 and an antenna 7 electrically connected to the PCB group 2.

Specifically, the illumination unit is fixed on one side of the illumination circuit board 210 facing the front enclosure 10, the camera unit 5 is fixed on one side of the image capturing and processing board 211 facing the front enclosure 10, two electrodes of the battery unit 6 are electrically connected to the power supply board 212 and the antenna transceiver board 213 respectively, and the antenna 7 is fixed on the antenna transceiver board 213.

The illumination circuit board 210 comprises a through hole 2100 for the camera unit 5 to pass through in the direction of the front enclosure 10. The camera unit 5 on the image capturing and processing board 211 passes through the through hole 2100 and a hollow portion of the annular illumination circuit board 210 in sequence and extends into the front enclosure 10.

The battery unit 6 is disposed between the power supply board 212 and the antenna transceiver board 213. The fixing structures further comprise battery fixing plates 64 respectively located at the positive and negative electrodes of the battery unit 6. The two battery fixing plates 64 are fixedly connected to the power supply board 212 and the antenna transceiver board 213, respectively. Specifically, they are fixedly connected through the positioning pins 124 and the positioning holes 125.

Further, the battery unit 6 comprises a plurality of batteries 60 connected in series, battery connectors 61 electrically connecting the adjacent batteries 60, and electrode connectors 62 connecting the positive and negative electrodes of the battery unit 6, and the two electrode connectors 62 are electrically connected to the power supply board 212 and the antenna transceiver board 213 respectively. The battery connectors 61 electrically connect a plurality of batteries 60 together to form a battery pack, and the electrode connectors 62 electrically connect the positive electrode or the negative electrode of the battery pack to a corresponding PCB for electrical output.

The battery 60 is a button cell. The battery unit 6 further comprises an insulation gasket 63 located around the negative electrode of the battery 60 to insulate the negative electrode from the positive electrode, and insulate the battery 60 from the battery connector 61 or the electrode connector 62 located at the insulation gasket 63.

The battery connector 61 is folded and located between two adjacent batteries 60 to save space, and the battery connector 61 can be connected to the battery 60 by means of welding or conductive adhesive, etc. The electrode connector 62 is fixed to the positive and negative electrodes of the battery unit 6 and the PCB preferably by welding, so as to improve stability. An end of the electrode connector 62 away from the battery 60 comprises a notch, and the corresponding power supply board 212 and the antenna transceiver board 213 comprise a notch 2120, which is convenient for wetting during soldering.

In addition, the medical capsule 100 further comprises a magnet unit 4 disposed between the image capturing and processing board 211 and the power supply board 212. The magnet unit 4 comprises a magnet and a magnet fixing cover 40 sleeved on the magnet.

The magnet fixing cover 40 is an internal hollow recess, and the height of the recess fits the magnet. Two sides of the magnet fixing cover 40 are fixedly connected to the image capturing and processing board 211 and the power supply board 212 through the positioning pins 124 and the positioning holes 125, respectively. Meanwhile, a dispensing hole 41 is set in the bottom of the magnet fixing cover 40. Since the magnet fixing cover 40 is large in size, glue is injected into the dispensing hole 41 to fix it to the adjacent PCB, so that the whole structure is more stable.

The PCB group 2 and the functional units of the present invention constitute an endoscope imaging module which is used for capturing images in the GI tract. The magnet unit 4 controls the movement and orientation of the medical capsule under the control of an external magnetic field, so as to capture images with better angles.

Referring to FIGS. 1 ~ 3, the pressure detection unit 3 is used for detecting the pressure in the GI tract, and comprises a pressure transmission film 30 mounted at the opening of the middle enclosure 11 away from the front enclosure 10, a pressure sensor 31 located in the middle enclosure 11 and cooperating with the pressure transmission film 30, and a pressure collecting and processing circuit board 32 electrically connected to the pressure sensor 31. The pressure collecting and processing circuit board 32 is connected to one of the PCBs through a flexible printed circuit 20. Preferably, the pressure collecting and processing circuit board 32 is connected to the antenna transceiver board 213 near the opening.

The pressure transmission film 30 is preferably made of silicone and comprises a pressure groove 301 which is concavely formed from inside to outside, a pressure film 302 arranged on the bottom surface of the pressure groove 301. The pressure sensor 31 is housed in the pressure groove 301 and faces the pressure film 302.

The pressure transmission film 30 further comprises an annular step 303 located at the upper end of the pressure groove 301. The pressure sensor 31 is fixed on the pressure collecting and processing circuit board 32 through a fixing seat 310 in a limiting fit with the annular step 303, so as to limit the distance between the pressure sensor 31 and the pressure film 302.

The pressure transmission film 30 further comprises a bonding part 304 which is concavely formed around the pressure transmission film 30. The bonding part 304 is bonded to the lower end of the middle enclosure 11 by glue, so that the pressure detection unit 3 and the middle enclosure 11 can be mounted more stably.

The cross section of the pressure groove 301 is T-shaped, and the thickness of the pressure film 302 located at the bottom of the pressure groove 301 is preferably 0.1mm-0.5mm.

The pressure collecting and processing circuit board 32 is fixed on one side of the supporting leg 13 away from the front enclosure 10, the pressure transmission film 30 is assembled on one end of the middle enclosure 11 away from the front enclosure 10, and the pressure sensor 31 is located between the pressure transmission film 30 and the pressure collecting and processing circuit board 32.

Further, the diameter of the pressure collecting and processing circuit board 32 is less than the diameter of other PCBs in the PCB group 2, and the pressure collecting and processing circuit board 32 is convexly provided with fixing legs 320 corresponding to the plurality of supporting legs 13 one by one, so as to facilitate the overall assembly. The assembly process that can be referenced is: the pressure collecting and processing circuit board 32 and the antenna transceiver board 213 are connected into a whole through the flexible printed circuit 20, and then the whole

PCB group 2 is inserted into the middle enclosure 11 from one end of the middle enclosure 11 close to the front enclosure 10, and the pressure collecting and processing circuit board 32 passes through gaps 130 between the supporting legs 13 to one side of the supporting legs 13 away from the front enclosure 10. Then, the fixing legs 320 are fixed to the supporting legs 13 with glue, etc.

To summarize, in the present invention, an endoscope imaging module is constituted by the PCB group 2 and the functional unit, and pressure detection of the GI tract is realized by the pressure detection unit 3, so that the functions of the medical capsule are diversified. In addition, the pressure detection unit 3 is disposed on the opening side of the enclosure, so that the internal structure of the medical capsule is reasonably distributed, the assembly is quick, and mass production is facilitated. Furthermore, the present invention has the feature of easy expansion and extension of functions. When different functions need to be added, it is convenient to expand the functions of the medical capsule by simply adding PCB, functional components, and corresponding fixing pieces in sequence.

It should be understood that, although the specification is described in terms of embodiments, not every embodiment merely comprises an independent technical solution. Those skilled in the art should have the specification, as a whole, and the technical solutions in each embodiment may also be combined as appropriate to form other embodiments that can be understood by those skilled in the art.

The series of detailed descriptions set forth above are only specific to a feasible embodiment of the present invention and are not intended to limit the scope of protection of the present invention, and any equivalent embodiments or variations made without departing from the spirit of the art of the present invention shall be included within the scope of protection of the present invention.

## Claims

1. A medical capsule comprising:
an enclosure, comprising an opening at one end along an axial direction;
a PCB group and a functional unit which are arranged inside the enclosure, wherein the PCB group comprises a plurality of PCBs which are connected through flexible printed circuits and are spaced from each other, and wherein the functional unit comprises a battery unit electrically connected to the PCB; and
a pressure detection unit, comprising a pressure transmission film assembled at the opening, a pressure sensor positioned in the enclosure, and a pressure collecting and processing circuit board electrically connected to the pressure sensor, wherein the pressure collecting and processing circuit board is connected to one of the PCBs through a flexible printed circuit.

2. The medical capsule of claim 1, wherein the pressure transmission film comprises a pressure groove which is concavely formed from inside to outside and a pressure film which is arranged on the bottom surface of the pressure groove, and the pressure sensor is housed in the pressure groove and faces the pressure film.

3. The medical capsule of claim 1, wherein the enclosure comprises a front enclosure and a middle enclosure adapted to each other, and a plurality of supporting legs are convexly fitted inside one end of the middle enclosure away from the front enclosure; and wherein the pressure collecting and processing circuit board is fixed on one side of the supporting legs away from the front enclosure, the pressure transmission film is assembled at one end of the middle enclosure away from the front enclosure, and the pressure sensor is positioned between the pressure transmission film and the pressure collecting and processing circuit board.

4. The medical capsule of claim 3, wherein the periphery of the pressure collecting and processing circuit board is convexly fitted with fixing legs corresponding to the plurality of supporting legs one by one.

5. The medical capsule of claim 1, wherein the enclosure comprises a front enclosure and a middle enclosure adapted to each other, and a fixing groove positioned at the joint of the front enclosure and the middle enclosure; and wherein
the PCB group further comprises fixing structures for connecting two adjacent PCBs, and the edge of one of the fixing structures is fixed in the fixing groove to fix the PCB group in the enclosure.

6. The medical capsule of claim 5, wherein the PCB group comprises an illumination circuit board, an image capturing and processing board, a power supply board and an antenna transceiver board which are sequentially arranged; and wherein the fixing structures comprise an illumination fixing structure, and the illumination circuit board is located in the illumination fixing structure; and
wherein the image capturing and processing board is fixed on one side of the illumination fixing structure away from the front enclosure, and the edge of the illumination fixing structure is housed in the fixing groove.

7. The medical capsule of claim 6, wherein the functional unit further comprises an illumination unit, a camera unit and an antenna, wherein the illumination unit is fixed on one side of the illumination circuit board facing the front enclosure, the camera unit is fixed on one side of the image capturing and processing board facing the front enclosure, the battery unit is electrically connected to the power supply board, and the antenna is fixed on the antenna transceiver board; and wherein the illumination fixing structure is annular, and the illumination circuit board is provided with a through hole for the camera unit to pass through towards the front enclosure.

8. The medical capsule of claim 6, wherein the illumination fixing structure comprises an annular retaining wall, a retaining frame protruding outwards from a front end of part of the retaining wall, and a plurality of fixing clamps protruding inwards from the retaining wall to fix and limit the illumination circuit board, wherein an edge of the retaining frame is fixed in the fixing groove; and wherein
the illumination circuit board is housed in a mounting groove formed by the surrounding of the retaining wall, and the image capturing and processing board is fixed to one side of the retaining wall away from the front enclosure.

9. The medical capsule of claim 6, wherein the battery unit is disposed between the power supply board and the antenna transceiver board, and the fixing structure further comprises battery fixing plates respectively located at the positive and negative electrodes of the battery unit, and the two battery fixing plates are fixedly connected to the power supply board and the antenna transceiver board respectively.

10. The medical capsule of claim 6, wherein the battery unit comprises a plurality of batteries connected in series, battery connectors electrically connected between the adjacent batteries, and electrode connectors connected to the positive and negative ends of the battery unit, and the two electrode connectors are electrically connected to the power supply board and the antenna transceiver board respectively.

11. The medical capsule of claim 6, wherein the medical capsule further comprises a magnet unit disposed between the image capturing and processing board and the power supply board, wherein the magnet unit comprises a magnet and a magnet fixing cover sleeved on the magnet, and the magnet fixing cover is fixedly connected to the image capturing and processing board and the power supply board.
